# EUROPEAN PATENT APPLICATION

(11) **EP 2 093 188 A2**
(43) Date of publication of application: **26.08.2009**
(21) Application number: 09152291.2
(22) Date of filing: 06.02.2009
(51) Int. Cl.: C01B 13/02

(54) **Method of generating an oxygen-enriched gas for a user**

(30) Priority: 22.02.2008 US 70975
(71) Applicant: Delphi Technologies, Inc., Troy, Michigan 48007 (US)
(72) Inventor: Chekal, Michael P., Brighton, MI 48116 (US); McClain, Michael S., Ortonville, MI 48462 (US); Pelletier, Dana G., Ortonville, MI 48462 (US); Voto, Andrew M., Brighton, MI 48116 (US)
(74) Representative: Denton, Michael John

(57) **Abstract**

A method of generating an oxygen-enriched gas for a user via an oxygen generating system is disclosed herein. The oxygen generating system includes at least one sieve bed having a nitrogen-adsorption material disposed therein, the nitrogen-adsorption material being configured to adsorb nitrogen from a feed gas introduced thereto, thereby generating the oxygen-enriched gas therefrom. The at least one sieve bed has an internal gas pressure within a volume defined by the at least one sieve bed. The method includes measuring the internal sieve bed pressure, measuring an ambient atmospheric parameter, and detecting inhalation of the user. The method further includes selectively controlling, substantially in real time, delivery of the oxygen-enriched gas to the user based on at least one of the internal sieve bed gas pressure measurement, the ambient atmospheric parameter measurement, the inhalation detection, or combinations thereof.

## Description

### TECHNICAL FIELD

The present disclosure relates generally to method(s) of generating an oxygen-enriched gas for a user.

### BACKGROUND OF THE INVENTION

Oxygen generating systems are often used to produce an oxygen-enriched gas for a user. Oxygen generating systems typically include a gas fractionalization system configured to separate oxygen from other components (e.g., nitrogen) in a feed gas to produce the oxygen-enriched gas. The gas fractionalization system, for example, may include one or more sieve beds having a nitrogen-adsorption material disposed therein and configured to adsorb at least nitrogen from the feed gas.

Many oxygen generating systems employ pulsed oxygen delivery, where a pulse of the oxygen-enriched gas generated by the sieve bed(s) is delivered to the user during fixed time intervals based on an inhalation detection of the user. These systems also generally use fixed valve timing based on a predetermined flow setting for delivery of each pulse.

### SUMMARY OF THE INVENTION

A method of generating an oxygen-enriched gas for a user via an oxygen generating system is disclosed herein. The oxygen generating system includes at least one sieve bed having a nitrogen-adsorption material disposed therein, the nitrogen-adsorption material being configured to adsorb nitrogen from a feed gas introduced thereto, thereby generating the oxygen-enriched gas therefrom. The at least one sieve bed has an internal gas pressure within a volume defined by the at least one sieve bed. The method includes measuring the internal sieve bed pressure, measuring an ambient atmospheric parameter, and detecting inhalation of the user. The method further includes selectively controlling, substantially in real time, delivery of the oxygen-enriched gas to the user based on at least one of the internal sieve bed gas pressure measurement, the ambient atmospheric parameter measurement, the inhalation detection, or combinations thereof.

### BRIEF DESCRIPTION OF THE DRAWINGS

Features and advantages of the present disclosure will become apparent by reference to the following detailed description and drawings, in which like reference numerals correspond to similar, though perhaps not identical components. For the sake of brevity, reference numerals or features having a previously described function may or may not be described in connection with other drawings in which they appear.

Fig. 1 is a schematic diagram of an example of an oxygen generating system;

Fig. 2 is a diagram depicting an example of a timing sequence of at least one valve of the system at a plurality of states of a cycle of an oxygen generating process; and

Fig. 3 is a diagram depicting another example of a timing sequence of at least one valve of the system at a plurality of states of a cycle of an oxygen generating process.

### DESCRIPTION OF THE PREFERRED EMBODIMENTS

Embodiment(s) of the method as disclosed herein advantageously employ real time methods of controlling valve timing to produce pulses of oxygen-enriched gas for a user. These pulses have a non-fixed duration and may be generated based on a demand from the user such as, e.g., an inhalation detection, a pressure measurement, and/or an ambient atmospheric parameter.

Example(s) of the method disclosed herein further advantageously provide the user with a pulse of the oxygen-enriched gas having a desirably high oxygen content. Further, the example(s) of the method substantially prevent a pulse of the oxygen-enriched gas from being delivered when the oxygen purity may be low and additionally optimizes the timing of the valves in the system to allow for the fastest possible breathing rate of the user.

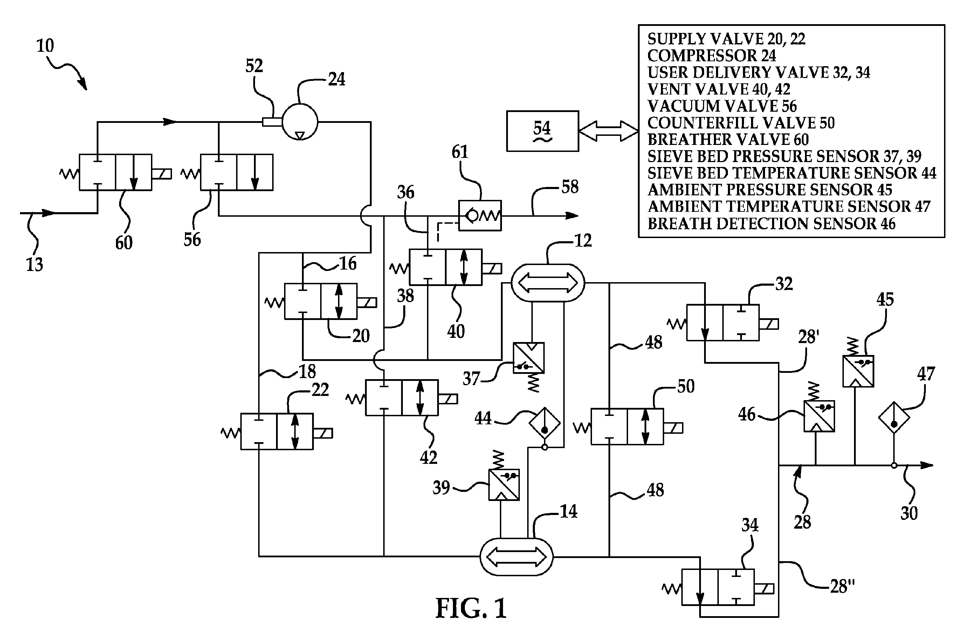

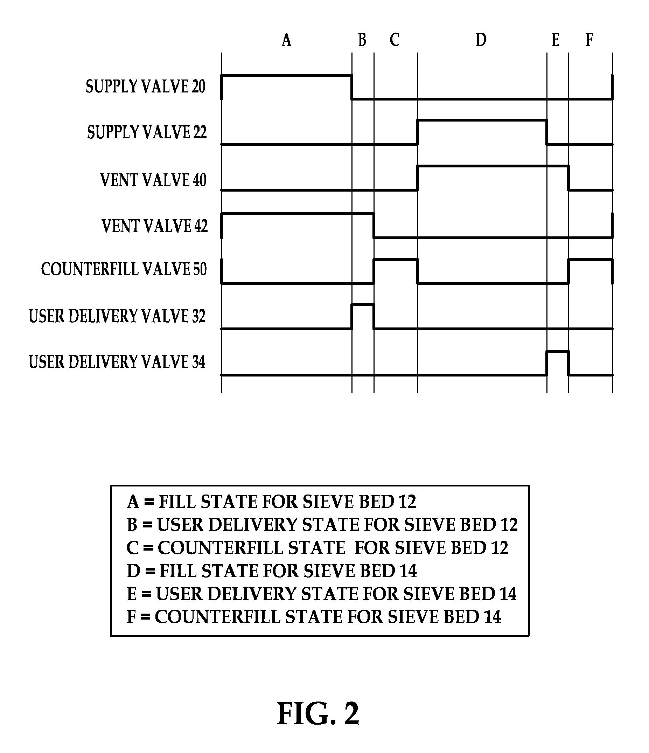

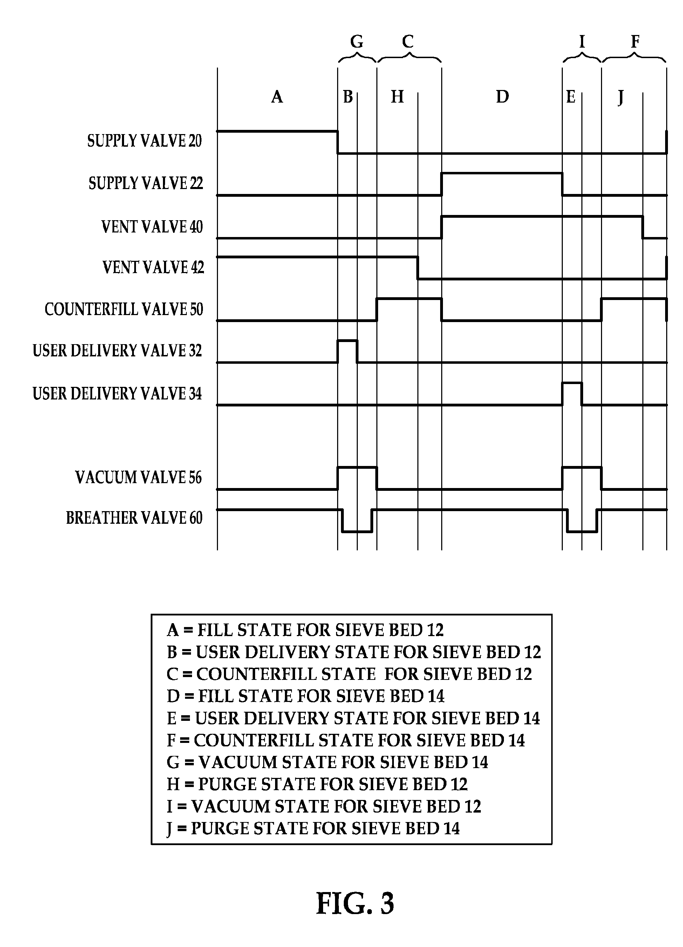

One non-limiting example of an oxygen generating system suitable for use with embodiment(s) of the method(s) and device(s) disclosed herein is depicted in Fig. 1. However, it is to be understood that any oxygen generating system may be suitable for use with the embodiment(s) of Figs. 2 and 3, various examples of which (not shown) are oxygen generating system(s) having fill valves (any suitable combination of 2-way, 3-way, 4-way valves, etc.), vent valves (any suitable combination of 2-way, 3-way, 4-way valves, etc.), a product tank(s), bleed orifice(s) and patient valving.

It is to be understood that the nitrogen-adsorption process employed by the oxygen generating system may be a pressure swing adsorption (PSA) process or a vacuum pressure swing adsorption (VPSA) process, and such processes operate in repeating adsorption/desorption cycles. In an embodiment of the present disclosure, each cycle includes at least a fill state, a user delivery state, and a counterfill state. In another embodiment of the present disclosure, each cycle further includes a vent state, a vacuum state, a purge state, a rest state, or combinations thereof.

The oxygen generating system 10 includes an inlet 13 configured to receive a feed gas. In a non-limiting example, the feed gas is air taken from the ambient atmosphere, which includes at least nitrogen, oxygen, and water vapor.

The oxygen generating device includes at least one sieve bed. In the example shown in Fig. 1, the oxygen generating device 10 includes first 12 and second 14 sieve beds, each in selective fluid communication with the feed gas. In an embodiment, each of the first 12 and second 14 sieve beds are configured to selectively receive the feed gas during a predetermined supply period. The first 12 and second 14 sieve beds may receive the feed gas via first 16 and second 18 supply conduits, respectively.

The first 16 and second 18 supply conduits are generally operatively connected to respective 20 and second 22 supply valves (or inlet valves). In a non-limiting example, the first 20 and second 22 supply valves are two-way valves. As provided above, the nitrogen-adsorption process employed by the oxygen generating device 10 operates via cycles, where one of the first 12 or second 14 sieve beds vents to atmosphere nitrogen-enriched (waste) gas, while the other of the first 12 or second 14 sieve beds delivers oxygen-enriched gas to the user. During the next cycle, the functions of the respective sieve beds 12, 14 switch. Switching is accomplished by opening the respective feed gas supply valve 20, 22 while the other of the feed gas supply valves 20, 22 is closed. More specifically, when one of the first 12 or second 14 sieve beds is receiving the feed gas, the respective one of the first 20 or second 22 supply valves is in an open position. In this case, the feed gas is prevented from flowing to the other of the first 12 or second 14 sieve beds. In an embodiment, the opening and/or closing of the first 20 and second 22 supply valves may be controlled with respect to timing of opening and/or closing and/or with respect to the sequence in with the first 20 and second 22 supply valves are opened and/or closed.

In an embodiment, the feed gas is compressed via, e.g., a compressor 24 prior to entering the first 16 or second 18 supply conduits. In a non-limiting example, the compressor is a scroll compressor. The compressor 24 includes a suction port 52 configured to draw in a stream of the feed gas from the inlet 13. In a non-limiting example, the suction port 52 is further operatively and selectively connected to the first 12 and second 14 sieve beds. In this configuration, the suction port 52 is configured to draw vacuum on the first 12 or second 14 sieve bed at pre-selected times during the adsorption/desorption cycle.

After receiving the compressed feed gas, the first 12 and second 14 sieve beds are each configured to separate at least most of the oxygen from the feed gas to produce the oxygen-enriched gas. In an embodiment, the first 12 and second 14 are each sieve beds 12, 14 including the nitrogen-adsorption material (e.g., zeolite, other similar suitable materials, and/or the like) configured to adsorb at least nitrogen from the feed gas.

In a non-limiting example, the oxygen-enriched gas generated via either the PSA or VPSA processes includes a gas product having an oxygen content ranging from about 70 vol% to about 95 vol% of the total gas product. In another non-limiting example, the oxygen-enriched gas has an oxygen content of at least 87 vol% of the total gas product.

A user conduit 28 having a user outlet 30 is in alternate selective fluid communication with the first and second sieve beds 12, 14. The user conduit 28 may be formed from any suitable material, e.g., at least partially from flexible plastic tubing. In an embodiment, the user conduit 28 is configured substantially in a "Y" shape. As such, the user conduit 28 may have a first conduit portion 28' and a second conduit portion 28", which are in communication with the first sieve bed 12 and the second sieve bed 14, respectively, and merge together before reaching the user outlet 30. The user outlet 30 may be an opening in the user conduit 28 configured to output the substantially oxygen-enriched gas for user use. The user outlet 30 may additionally be configured with a nasal cannula, a respiratory mask, or any other suitable device, as desired.

In an embodiment, as shown in Fig. 1, the oxygen delivery device 10 also includes a sieve bed pressure sensor 37, 39 for the sieve beds 12, 14, respectively, and a sieve bed temperature sensor 44 configured to measure the pressure and temperature, respectively, of the first 12 and second 14 sieve beds during the PSA process. In another embodiment, a single pressure sensor may be used to measure the pressure of each of the sieve beds 12, 14, whereby the delivery device 10 may include additional equipment used for selecting the desired sieve bed 12, 14 that the pressure sensor is intended to measure. The device 10 further includes an ambient pressure sensor 45 and an ambient temperature sensor 47 to measure the pressure and temperature, respectively, of the ambient environment. Although sensors 45 and 47 are schematically shown inline with the user output 30, it is to be understood that these sensors may be placed in any suitable location so as to achieve readings with desirable accuracy.

The first conduit portion 28' and the second conduit portion 28" may be configured with a first user delivery valve 32 and a second user delivery valve 34, respectively. In an embodiment, the first 32 and the second 34 user delivery valves are configured as two-way valves. It is contemplated that when the oxygen-enriched gas is delivered from one of the first and second sieve beds 12, 14, to the user conduit 28, the respective one of the first 32 or second 34 user valves is open. Further, when the respective one of the first 32 or second 34 user valves is open, the respective one of the first 20 or second 22 feed gas supply valves is closed.

The nitrogen-adsorption process selectively adsorbs at least nitrogen from the feed gas. Generally, the compressed feed gas is introduced into one of the first 12 or the second 14 sieve beds, thereby pressurizing the respective first 12 or second 14 sieve bed. Nitrogen and possibly other components present in the feed gas are adsorbed by the nitrogen-adsorption material disposed in the respective first 12 or second 14 sieve bed during an appropriate PSA/VPSA cycle. After: a predetermined amount of time; reaching a predetermined target pressure; detection of an inhalation; and/or another suitable trigger, the pressure of the respective first 12 or second 14 sieve bed is released. At this point, the nitrogen-enriched gas (including any other adsorbed components) is also released from the respective first 12 or second 14 sieve bed and is vented out of the system 10 through a main vent conduit 58. As shown in Fig. 1, the nitrogen-enriched gas in the first sieve bed 12 is vented through a vent port/conduit 36 for the first sieve bed 12 when a first vent valve 40 is open, and the nitrogen-enriched gas in the second sieve bed 14 is vented through a vent conduit 38 for the second sieve bed 14 when a second vent valve 42 is open. The vent conduits 36 and 38 merge into the main vent conduit 58. It is to be understood that venting occurs after each dynamically adjusted oxygen delivery phase and after counterfilling, each of which will be described further below. The gas not adsorbed by the nitrogen-adsorption material (i.e., the oxygen-enriched gas) is delivered to the user through the user outlet 30.

In an embodiment, the oxygen delivery system 10 may be configured to trigger an output of a predetermined volume of the oxygen-enriched gas from the sieve bed 12 upon detection of an inhalation by the user. Detection of an inhalation may be accomplished via, e.g., a breath detection device, schematically shown as reference numeral 46 in Fig. 1. The predetermined volume, which is at least a portion of the oxygen-enriched gas produced, is output through the user conduit 28 and to the user outlet 30 during a respective dynamically adjusted oxygen delivery phase.

As used herein, a "masked" time or the like language may be defined as follows. Following a dynamically adjusted user oxygen delivery phase from the first 12 or second 14 sieve bed, breath detection may be "masked" for a predetermined masking time, for example, during the dynamically adjusted oxygen delivery phase and during a predetermined amount of time following the delivery phase. It is understood that such predetermined masking time may be configured to prevent the triggering of another dynamically adjusted user oxygen delivery phase before sufficient substantially oxygen-enriched gas is available from the other of the second 14 or first 12 sieve bed. As used herein, sufficient substantially oxygen-enriched gas may be a pulse having a desired oxygen content. In an embodiment, the predetermined masking time may be short in duration. As a non-limiting example, the predetermined masking time may be about 500 milliseconds in length. In an alternate embodiment, this masking time may also be dynamically adjusted, e.g., based on the average breath rate. Further, in order to accommodate a maximum breathing rate of 30 Breaths Per Minute (BPM), a maximum mask time of 2 seconds may be used, if desired.

The first 12 and second 14 sieve beds are also configured to transmit at least a portion of the remaining oxygen-enriched gas (i.e., the oxygen-enriched gas not delivered to the user during or after the masked time to the user outlet 30), if any, to the other of the first 12 or second 14 sieve bed. This also occurs after each respective dynamically adjusted oxygen delivery phase. The portion of the remaining oxygen-enriched gas may be transmitted via a counterfill flow conduit 48. The transmission of the remaining portion of the oxygen-enriched gas from one of the first 12 or second 14 sieve beds to the other first 12 or second 14 sieve beds may be referred to as "counterfilling."

As shown in Fig. 1, the counterfill flow conduit 48 may be configured with a counterfill flow valve 50. In a non-limiting example, the counterfill flow valve 50 is a two-way valve. The counterfill flow valve 50 is opened to allow the counterfilling of the respective first 12 and second 14 sieve beds.

The compressor 24, the first 20 and second 22 supply valves, the first 32 and second 34 user delivery valves, and the first 40 and second 42 vent valves are controlled by a controller 54. The sieve bed pressure sensors 37, 39, and the sieve bed temperature sensor 44 measure internal system parameters, and the ambient pressure sensor 45 and the ambient temperature pressure sensor 47 measure ambient atmospheric parameters, all of which are inputs to the controller 54. In a non-limiting example, the controller 54 is a microprocessor including a memory. As will be described in more detail below, the controller 54 receives, e.g., sieve bed pressures, and other similar variables, and uses these variables to execute one or more algorithms for controlling various components and/or processes used in the system 10.

In an embodiment, the oxygen generating system 10 may further include a vacuum valve 56 operatively connected to the main vent conduit 58 and in operative and selective fluid communication with the suction port 52 of the compressor 24 via the inlet line 13. The vacuum valve 56 assists the suction port 52 in drawing at least the feed gas from the sieve beds 12, 14 during a vacuum state of the adsorption/desorption cycle, as will be described in more detail below.

In some instances, the oxygen generating system 10 may further include a check valve 61 operatively disposed on the main vent conduit 58. The check valve 61 is configured to prevent air from the atmosphere being pulled into the system 10 via the main vent conduit 58 when the vacuum valve 56 is open and a vacuum is applied to the sieve beds 12, 14.

The oxygen generating system 10 may also include a breather valve 60 operatively connected to the inlet 13. The breather valve 60 generally assists in allowing the feed gas, taken from the ambient atmosphere, to be directed to the suction port 52 of the compressor 24.

A method of generating an oxygen-enriched gas for a user via the oxygen generating system 10 includes: measuring the internal sieve bed gas pressure; measuring an ambient atmospheric pressure; detecting inhalation of the user; and selectively controlling, substantially in real time, delivery of the oxygen-enriched gas to the user based on at least one of the internal sieve bed gas pressure measurement, the ambient atmospheric parameter measurement, the inhalation detection, or combinations thereof.

With reference now to Figs. 1 and 2, each adsorption/desorption cycle of the nitrogen-adsorption process includes at least a fill state (fill states A and D), a user delivery state (user delivery states B and E), and a counterfill state (counterfill states C and F) for each of the sieve beds 12, 14. In an embodiment, the fill state of the sieve bed 12 (fill state A) begins after the counterfill state of the sieve bed 14 (counterfill state F) of a previous adsorption/desorption cycle. For purposes of illustration, the counterfill state of the previous adsorption/desorption cycle includes transmission of oxygen-enriched gas in the second sieve bed 14 to the first sieve bed 12, the amount of which remains after a pulse of the gas has been delivered to the user. During the previous counterfill state, the supply valve 22 and the user delivery valve 34 of the second sieve bed 14 are closed, as well as the vent valve 40 and the user delivery valve 32 of the first sieve bed 12.

During the fill state A, the method includes opening the supply valve 20 of the first sieve bed 12 to supply the feed gas to the first sieve bed 12, and opening the vent valve 42 of the second sieve bed 14 to vent or purge at least a portion of the nitrogen (i.e., the nitrogen-enriched gas) from the second sieve bed 14.

Also during the fill state A, the first sieve bed 12 is pressurized to a target pressure (P_{T}) as the first sieve bed 12 is supplied with the feed gas. The target pressure (P_{T}) is generally determined for each adsorption/desorption cycle. In a non-limiting example, the target pressure (P_{T}) is based on at least a flow setting of the oxygen generating device 10, the ambient temperature, and the ambient pressure. Details of an example of a suitable method of determining the target pressure (P_{T}) may be found in U.S. Provisional Application Ser. No. , filed concurrently herewith (Docket No. DP-317406), which is commonly owned by the Assignee of the present disclosure, and is incorporated herein by reference in its entirety.

It is to be understood that, to achieve the desired oxygen purity, the pressure of the first sieve bed 12 is substantially equal to the pressure of the second sieve bed 14. It is to be understood that this pressure equilibrium between the first 12 and the second 14 sieve beds is achieved at substantially the same pressure as the target pressure (P_{T}). Without being bound to any theory, it is believed that having the pressure equilibrium between the sieve beds 12, 14 and the target pressure (P_{T}) substantially the same allows for desirable operation of the compressor 24, sufficient production of the oxygen-enriched gas, and sufficient removal of the nitrogen-enriched gas from the system 10. In an embodiment, the pressure equilibrium between the first 12 and the second 14 sieve beds is achieved by controlling the speed (e.g., controlling a pulse width modulation (PWM) setting) of the compressor 24. Changes to the PWM setting for each fill state (e.g., the fill states A and D) are based on a pressure difference between the target pressure P_{T} of the first sieve bed 12 and a peak pressure of the first sieve bed 12 determined from the previous fill state A.

It is to be understood that the PWM setting of the compressor 24 may also be controlled based on an inhalation detection of the user. If, for example, an inhalation is not detected by 1) the time the target pressure P_{T} of the sieve bed 12 is reached, and/or 2) a predetermined time limit, the fill state A may be temporarily stopped until the next inhalation detection. While the fill state A is stopped, the internal pressure of the sieve bed 12 is substantially maintained. Also, while the fill state A is stopped, the compressor 24 may also be stopped or at least the motor driving the compressor 24 may be throttled down to a lower power. In this case, all of the valves, except the breather valve 60, (i.e., the supply valves 20, 22, the user delivery valves 32, 34, the vent valves 40, 43, and the counterfill valve 50) are closed.

After the fill state A is substantially complete and after an inhalation detection of the user, the user delivery state B begins. It is to be understood that the inhalation detection may be masked for a small interval of time (as described above) to prevent activation of the use delivery state B before enough oxygen-enriched gas is available for the user from the sieve bed 14.

During the user delivery state B, the user delivery valve 32 for the first sieve bed 12 opens and the oxygen-enriched gas generated by the sieve bed 12 flows to the user delivery conduit 28. Also, the supply valve 22 for the second sieve bed 14 opens so that the feed gas may be supplied to the sieve bed 14.

The duration of the user delivery state B is determined for each adsorption/desorption cycle of the nitrogen-adsorption process. In a non-limiting example, the duration of the user delivery state B is based on at least one of a calibration value of the supply valve 20, a calibration value of the supply valve 22, a calibration value of the user delivery valve 32, a calibration value of the user delivery valve 34, a calibration value of the vent valve 40, a calibration value of the vent valve 42, a flow setting for the feed gas, a pressure of the sieve bed 12, the ambient temperature, the ambient pressure, a breathing rate of the user, or combinations thereof. Details of an example of a suitable method for how the duration of the user delivery state B is determined may be found in U.S. Provisional Application Ser. No. , filed concurrently herewith (Docket No. DP-317407), which is commonly owned by the Assignee of the present disclosure, and is incorporated herein by reference in its entirety.

The counterfill state C begins after the user delivery state B. In the counterfill state C, the method includes opening the counterfill valve 50, closing the supply valve 20 and the user delivery valve 32 for the first sieve bed 12, and closing the supply valve 22 and the user delivery valve 34 for the second sieve bed 14. In a non-limiting example, the counterfill state C occurs until the pressure between the first 12 and the second 14 sieve beds is substantially equal.

In another embodiment, the method further includes selectively applying vacuum to the first sieve bed 12 during delivery of the oxygen-enriched gas to the user. In a non-limiting example, the vacuum is selectively applied to the first sieve bed 12 via the suction port 52 of the compressor 24, the details of which will be described if further detail below.

With reference now to Figs. 1 and 3, the vacuum is selectively applied during a vacuum state (i.e., the vacuum state G) of the adsorption/desorption cycle. The vacuum state occurs during the user delivery state B for the sieve bed 12, and after or during venting of sieve bed 14. During the vacuum state G, the method includes opening the vacuum valve 56. The breather valve 60 is closed for at least a portion of the vacuum state G. In a non-limiting example, the breather valve 60 is open for a relatively short period of time (e.g., 55 mS) at the start of the vacuum state G and the end of the vacuum state G to substantially prevent the compressor 25 from blocking the outflow of the compressed feed gas, a phenomenon often referred to as "deadheading".

The vacuum state generally occurs for a time period based on the target pressure P_{T} of the first sieve bed 12 determined after each inhalation detection. In a non-limiting example, the vacuum state occurs for a time period spanning between pressurization and depressurization of the sieve bed 12 (i.e., the time between the fill state D and the user delivery state E). The time of the vacuum state may be determined as a function of sieve pressure.

In yet another embodiment, the adsorption/desorption cycle further includes a purge state (i.e., the purge state H). During the purge state, a portion of the pressurized oxygen-enriched gas produced in the sieve bed 14 is delivered to the other sieve bed 12 to substantially clean the sieve bed 12 for another adsorption/desorption cycle. The purge state H begins after the vacuum state G and substantially simultaneously with the counterfill state C. During the purge state H, the method includes closing the vacuum valve 56, opening the counterfill valve 50 and vent valve 42, and purging the first sieve bed 12. In a non-limiting example, the purge state H occurs for a time period based on a calibration value of the vent valve 40, a purge volume calibration value, the internal pressure of the sieve bed 12 at the start of the purge state H, the ambient pressure, and the ambient temperature. The length of the time of the purge state may be determined in a manner similar to that disclosed for determining the patient time to generate a gas bolus as provided in U.S. Provisional Ser. No. (Docket No. DP-317407), as referenced above. In a non-limiting example, the time for a purge state may range from about 50 ms to about 300 ms.

As used herein, "venting" releases a pressurized sieve bed 12 to atmosphere. In embodiment(s) of the present disclosure, vacuum may then or simultaneously be applied to that same sieve bed 12 to pull the pressure of the sieve bed 12 down further (e.g., substantially at or below atmospheric levels). During the vent (and vacuum, if used) state, waste (e.g. nitrogen-enriched) gas is expelled from the sieve bed 12. As used herein, "purging" takes a predetermined amount of pressurized oxygen-enriched gas from the other sieve bed 14 and blows it through the vented (and vacuumed) sieve bed 12 to aid in preparing sieve bed 12 for new production of oxygen-enriched gas.

In instances where the target pressure P_{T} of the sieve bed 12 is reached before an inhalation is detected, the adsorption/desorption cycle may enter a rest state (not shown). During the rest state, the user delivery valves 32, 34, the supply valves 20, 22, the counterfill valve 50, and the vacuum valve 56 are closed, and the breather valve 60 is opened.

Once the counterfill state C is complete (i.e., the counterfill state for the sieve bed 12), the fill state D for the sieve bed 14 begins. The adsorption/desorption cycle continues for at least the fill state D, the user delivery state E, and the counterfill state F of the second sieve bed 14. In some embodiments, the cycle further includes a vacuum state I, a vent state J, a purge state H, and possibly the rest state. It is to be understood that the method described above repeats itself for each complete adsorption/desorption cycle. For example, the cycle ends when the counterfill state F is complete for the sieve bed 14, and then a new cycle begins starting with the fill state A for the sieve bed 12.

It is to be understood that, in the embodiments of the method provided above, the feed gas is taken from the ambient atmosphere and, in some instances, the feed gas includes water. If water is present in the feed gas when the feed gas is introduced to the sieve beds 12, 14, the water may degrade or possibly deactivate the nitrogen-adsorption material disposed in the sieve beds 12, 14. This degradation and/or deactivation may, in some instances, deleteriously affect the nitrogen-adsorption process and produce an oxygen-enriched gas potentially having a lower oxygen content than desired.

It is further to be understood that embodiments of the method may be applied to both stationary and portable oxygen generating systems. Particularly for portable applications, it is advantageous to reduce the weight of the system, as well as its size (in terms of volume), as compared to other stationary oxygen generating systems. In a non-limiting example, the size of the oxygen generating system 10 ranges from about 100 in³ to about 1500 in³, and the weight of the system 10 ranges from about 1 1b to about 20 lbs.

One way of removing the water adsorbed by the nitrogen-adsorbing material (e.g., zeolite) is to apply a vacuum to the sieve beds 12, 14, such as the vacuum states G and I in some of the embodiments described above. Rather than using a vacuum pump in the system 10, the size and weight of the oxygen generating system 10 may be further reduced by using the suction port 52 of the compressor 24 to apply the vacuum to the sieve beds 12, 14. The application of the vacuum to the sieve beds 12, 14 generally occurs during the vacuum state G and I and substantially simultaneously with, or after the venting state of the methods described above. The venting states for sieve beds 12, 14 occur at the same time that the other sieve bed 14, 12 is filling (i.e., the venting state for sieve bed 12 will be the same as state D, and the venting state for sieve bed 14 will be the same as state A). It is to be understood that if the vacuum is applied to the sieve beds 12, 14 during another state, it may be possible to overload the compressor 24 and potentially damage it. Overloading the compressor 24 may also cause substantially higher power consumptions of the system 10 as a whole, thereby wasting power. To reduce overloading the compressor 24, the vacuum is applied during the time between, e.g., when the sieve beds 12, 14 are pressurized and depressurized in the adsorption/desorption cycle, as provided above.

With reference again to Fig. 1, the suction port 52 is in operative, fluid communication with the first 12 and second 14 sieve beds via the main vent conduit 58. For example, a portion of the waste gas may be pulled from the sieve bed 12 via the suction port 52 and opening the vacuum valve 56. The waste gas pulled by the suction port 52 flows from the sieve bed 12, through the vent conduit 36, and into the main vent conduit 58. The check valve 61 disposed on the main vent conduit 58 substantially ensures that the vacuum is directed to the sieve beds 12, 14 so that air from the ambient atmosphere is not pulled into the system 10 through the main vent conduit 58. It is to be understood that the vacuum applied to the sieve beds 12, 14 assists in removing and/or venting the nitrogen-enriched gas from the sieve bed 12.

It is to be understood that the terms "connect/connected" and "engage/engaged" are broadly defined herein to encompass a variety of divergent connection and engagement arrangements and assembly techniques. These arrangements and techniques include, but are not limited to (1) the direct connection or engagement between one component and another component with no intervening components there between; and (2) the connection or engagement of one component and another component with one or more components there between, provided that the one component being "connected to" or "engaged to" the other component is somehow operatively connected to the other component (notwithstanding the presence of one or more additional components there between).

While several embodiments have been described in detail, it will be apparent to those skilled in the art that the disclosed embodiments may be modified and/or other embodiments may be possible. Therefore, the foregoing description is to be considered exemplary rather than limiting.

## Claims

1. A method of generating an oxygen-enriched gas for a user via an oxygen generating system, the oxygen generating system including at least one sieve bed having a nitrogen-adsorption material disposed therein, the nitrogen-adsorption material being configured to adsorb nitrogen from a feed gas introduced thereto, thereby generating the oxygen-enriched gas therefrom, the at least one sieve bed having an internal gas pressure within a volume defined by the at least one sieve bed, the method comprising:
measuring the internal sieve bed gas pressure;
measuring an ambient atmospheric parameter;
detecting inhalation of the user; and
selectively controlling, substantially in real time, delivery of the oxygen-enriched gas to the user based on at least one of the internal sieve bed gas pressure measurement, the ambient atmospheric parameter measurement, the inhalation detection, or combinations thereof.

2. The method as defined in claim 1 further comprising selectively applying vacuum to the at least one sieve bed during the delivery of the oxygen-enriched gas to the user.

3. The method as defined in claim 1 or claim 2 wherein the ambient atmospheric parameter is at least one of ambient atmospheric pressure or ambient atmospheric temperature.

4. The method as defined in claim 3 wherein the at least one sieve bed includes a first sieve bed and a second sieve bed, each of the first and second sieve beds including a respective supply valve, user delivery valve, and vent valve,
wherein the oxygen generating system further includes a counterfill valve, and
wherein the oxygen-enriched gas is generated during a cycle of the nitrogen-adsorption process in the first and second sieve beds, the cycle including at least a fill state, a counterfill state, and a user delivery state.

5. The method as defined in claim 4 wherein the fill state begins after the counterfill state of a previous cycle, and wherein during the fill state, the method further comprises:
opening the supply valve of the first sieve bed to supply the first sieve bed with the feed gas;
opening the vent valve of the second sieve bed to vent at least a portion of the adsorbed nitrogen from the second sieve bed; and
pressurizing the first sieve bed to a target pressure as the first sieve bed is supplied with the feed gas.

6. The method as defined in claim 5 wherein the target pressure is determined for each cycle of the nitrogen-adsorption process, and wherein the determination is based on a calibration of the oxygen generating system, a flow setting of the feed gas, the ambient temperature, and the ambient pressure.

7. The method as defined in claim 5 wherein the first sieve bed is also pressurized until a pressure equilibrium between the first and second sieve beds is substantially achieved.

8. The method as defined in claim 7 wherein the oxygen generating system further includes a compressor for compressing the feed gas to be supplied to at least one of the first and the second sieve beds, and wherein the pressure equilibrium between the first and second sieve beds is substantially achieved by controlling a speed of the compressor.

9. The method as defined in claim 8 wherein the speed of the compressor is controlled based on a pressure difference between the target pressure and a peak pressure determined from the fill state of a previous cycle.

10. The method as defined in claim 9 wherein the speed of the compressor is further controlled based on an inhalation detection of the user.

11. The method as defined in claim 6 wherein the user state begins after the fill state and after an inhalation detection of the user, and wherein during the user state, the method further comprises opening the supply valve for the second sieve bed and the user delivery valve for the first sieve bed.

12. The method as defined in claim 11 wherein a duration of the user state is determined for each cycle of the nitrogen-adsorption process, the duration being based on at least one of: a calibration value of at least one of the user delivery valve for the first sieve bed, of the user delivery valve for the second sieve bed; a flow setting for the feed gas; a pressure of the first sieve bed; the ambient temperature; the ambient pressure; a breathing rate of the user; or combinations thereof.

13. The method as defined in claim 11 wherein the inhalation detection is masked for a period of time, thereby substantially preventing activating the user state before a sufficient amount of oxygen-enriched gas is available for the user.

14. The method as defined in claim 11 wherein the counterfill state begins after the user state, and wherein the method further comprises:
opening the counterfill valve;
closing the supply valve and the user delivery valve for the first sieve bed; and
closing the supply valve and the user delivery valve for the second sieve bed.

15. The method as defined in claim 14 wherein the oxygen generating system further includes a vacuum valve and a breather valve, each of which are operatively connected to an inlet of the oxygen generating system, and wherein the cycle for the nitrogen-adsorption process further includes a vent state, a vacuum state, a purge state, a rest state, or combinations thereof.

16. The method as defined in claim 15 wherein the vacuum state occurs during the user state and during or after the vent state, and wherein during the vacuum state, the method further comprises:
opening the vacuum valve; and
closing the breather valve for at least a portion of the vacuum state;
wherein the vacuum state occurs for a time period based on a target pressure of the first sieve bed determined after each inhalation detection.

17. The method as defined in claim 16 wherein the purge state occurs substantially simultaneously with the counterfill state, and wherein during the purge state, the method further comprises:
closing the vacuum valve;
opening the counterfill valve and the vent valve of the first sieve bed; and
purging the first sieve bed;
wherein the purge state occurs for a time period based on a calibration value of the vent valve of the first sieve bed, a purge volume calibration value, the sieve bed pressure at the start of the purge state, the ambient pressure, and the ambient temperature.

18. The method as defined in claim 15 wherein the rest state occurs when the target pressure of the first sieve bed is reached before an inhalation detection, and wherein during the rest state, the method further comprises:
closing the user delivery valve for the first and second sieve beds, the supply valve for the first and second sieve beds, the vent valve for the first and second sieve beds, the counterfill valve, and the vacuum valve; and
opening the breather valve.

19. A method as defined in claim 2 wherein the feed gas is compressed via a compressor, and wherein the selective application of a vacuum is via a suction port of the compressor.

20. The method as defined in claim 19 wherein the oxygen-enriched gas is generated during a cycle of the nitrogen-adsorption process in the at least one sieve bed, the cycle including at least a user state, and wherein during the user state, the at least one sieve bed is substantially completely depressurized.

21. The method as defined in claim 20 wherein the vacuum is selectively applied during the user state of the nitrogen-adsorption process cycle.

22. The method as defined in claim 21 wherein the oxygen-enriched gas generating cycle further includes a fill state, and wherein during the fill state, the at least one sieve bed is pressurized.

23. The method as defined in claim 22 wherein the vacuum is selectively applied for a period of time between the user state and the fill state of the nitrogen-adsorption cycle.

24. The method as defined in claim 19 wherein selectively applying vacuum to the at least one sieve bed via the suction port assists in venting at least waste gas from the at least one sieve bed.

25. An oxygen generating system, comprising:
an inlet configured to receive a feed gas including at least nitrogen, oxygen, and water;
at least one sieve bed configured to generate an oxygen-enriched gas for a user by adsorbing nitrogen from the feed gas via a nitrogen-adsorption process;
at least one pressure sensor operatively connected to the at least one sieve bed and configured to measure an internal sieve bed gas pressure;
at least one valve in selective fluid communication with the at least one sieve bed and configured to regulate delivery of the oxygen-enriched gas to the user;
at least one sensor operatively connected to the oxygen generating system and configured to measure at least one of an ambient atmospheric pressure or an ambient atmospheric temperature; and
a compressor including a suction port, wherein the suction port is operatively and selectively connected to the at least one sieve bed.

26. The oxygen generating system as defined in claim 25 wherein the suction port is configured to selectively draw vacuum on the at least one sieve bed during the delivery of the oxygen-enriched gas to the user.

27. The oxygen generating system as defined in claim 26 wherein the vacuum selectively drawn by the suction port is configured to assist in venting at least waste gas from the at least one sieve bed.

28. The oxygen generating system as defined in claim 25 wherein the suction port is operatively connected to a first conduit configured to pull the feed gas from the ambient atmosphere into the compressor, and is operatively connected to a second conduit configured to pull at least a portion of the waste gas from the at least one sieve bed.

29. The oxygen generating system as defined in claim 28 wherein the first and second conduits are configured with a vacuum valve and a breather valve, respectively.

30. The oxygen generating system as defined in claim 28 wherein the at least one sieve bed further includes a vent port, and wherein the second channel is in selective fluid communication with the vent port, whereby when the at least a portion of the waste gas is pulled from the at least one sieve bed, the at least a portion of the waste gas is vented from the oxygen generating system.
